# EUROPEAN PATENT SPECIFICATION

(11) **EP 4 309 563 B1**
(45) Date of publication and mention of the grant of the patent: **12.02.2025**
(21) Application number: 23187041.1
(22) Date of filing: 21.07.2023
(51) Int. Cl.: A61B 1/00

(54) **ENDOSCOPIC MEDICAL DEVICE, IN PARTICULAR FOR COLONOSCOPY**
ENDOSKOPISCHES MEDIZINISCHES GERÄT, INSBESONDERE FÜR DIE KOLONOSKOPIE
DISPOSITIF MÉDICAL ENDOSCOPIQUE, EN PARTICULIER POUR COLOSCOPIE

(30) Priority: 22.07.2022 IT 202200015456
(43) Date of publication of application: 24.01.2024
(73) Proprietor: Prmedical S.r.l., 30174 Venezia (VE) (IT)
(72) Inventor: Podda, Riccardo, 30174 Venezia (VE) (IT)
(74) Representative: Gallo, Luca

(56) References cited:
- WO-A1-2016/210306
- CN-U- 207 870 874

## Description

### Field of the invention

The present invention relates to an endoscopic medical device, in particular for colonoscopy.

### State of the art

Various prior art solutions provide for colonoscopy devices which set out to increase the adenoma detection rate (ADR) during endoscopic procedures, especially for endoscopic screening.

To this end, during endoscopic examination, there arises the need to thoroughly inspect colon in all its parts, checking behind each curve and each single mucous membrane fold.

The problem raises from the fact that such inspection is long and complex if carried out appropriately, it requires multiple maneuvers by the operator and these maneuvers do not always allow to stretch the colon in a sensitive, non-traumatic and efficient manner.

It is clear that the need to stretch fully is diametrically opposed to that of stretching in a non-traumatic fashion.

Therefore, there arises the need for providing devices that are simultaneously capable of obtaining a complete and non-traumatic screening.

Up to date, prior art solutions are not able to meet this need; as a matter of fact, they are complex, traumatic and make the procedure longer and more complicated.

Prior art documents WO2016/210306 and CN207870874 disclose each a similar device to the one claimed by the invention. However, they are structurally different.

### Summary of the invention

Therefore, there is felt the need to overcome the drawbacks and limitations mentioned with reference to the prior art.

Such need is met by an endoscopic medical device according to claim 1.

### Description of the drawings

Further characteristics and advantages of present the invention will be more apparent from the description outlined below regarding the preferred and non-limiting examples thereof, wherein:

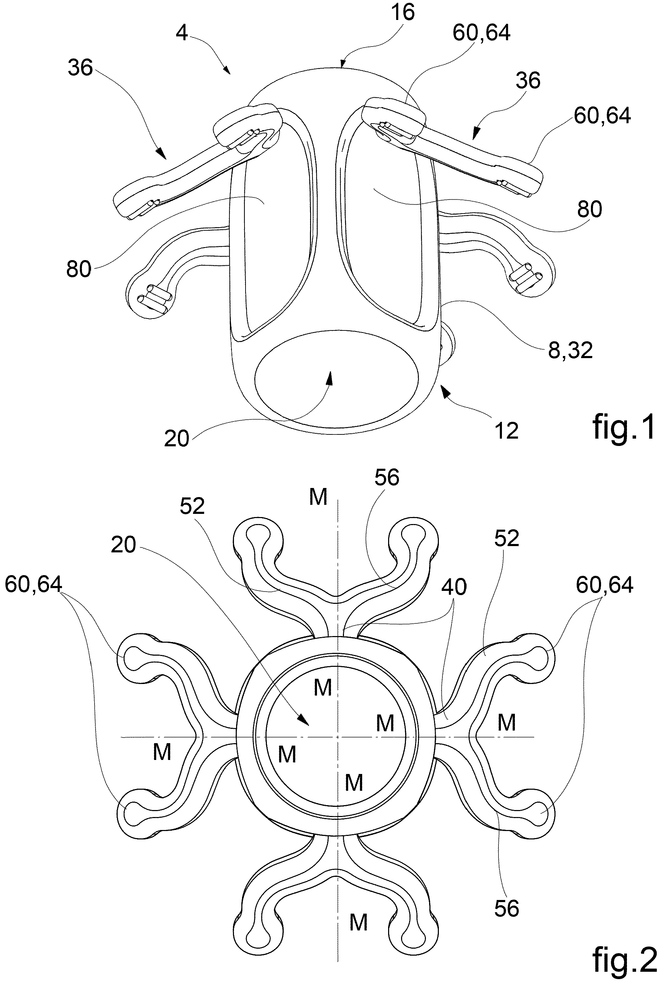

figure 1 shows a front perspective view of an endoscopic medical device, according to an embodiment of the present invention;
figure 2 shows a top plan view of the endoscopic medical device of figure 1;

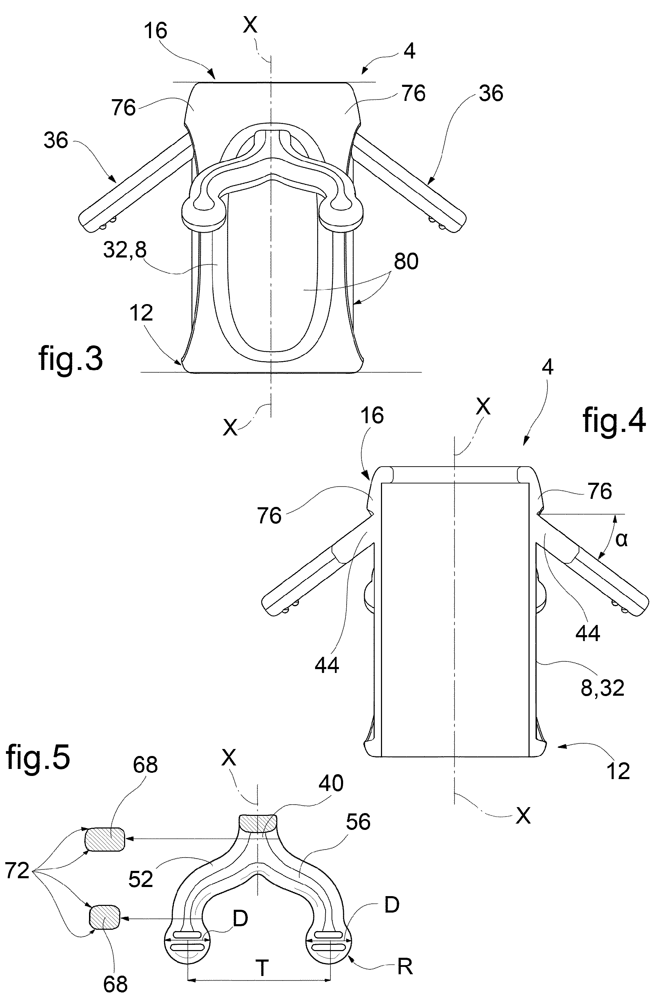

figure 3 shows a lateral view of the endoscopic medical device of figure 1;
figure 4 shows a cross-sectional view of the endoscopic medical device of figure 1;
figure 5 shows some enlarged details, even cross-sectional, of the endoscopic medical device of figure 1;

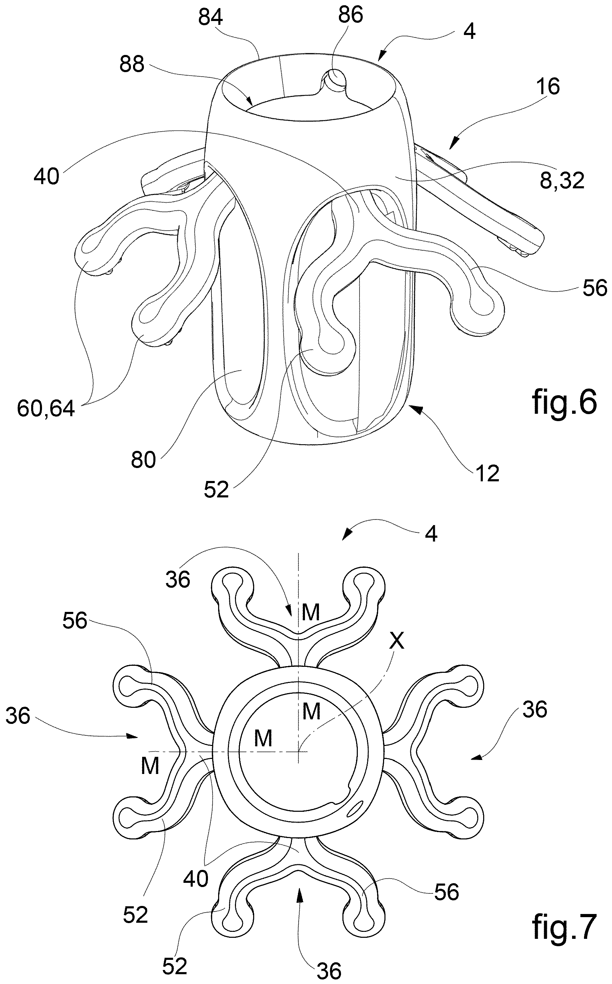

figure 6 shows a front perspective view of an endoscopic medical device, according to a further embodiment of the present invention;
figure 7 shows a top plan view of the endoscopic medical device of figure 6;

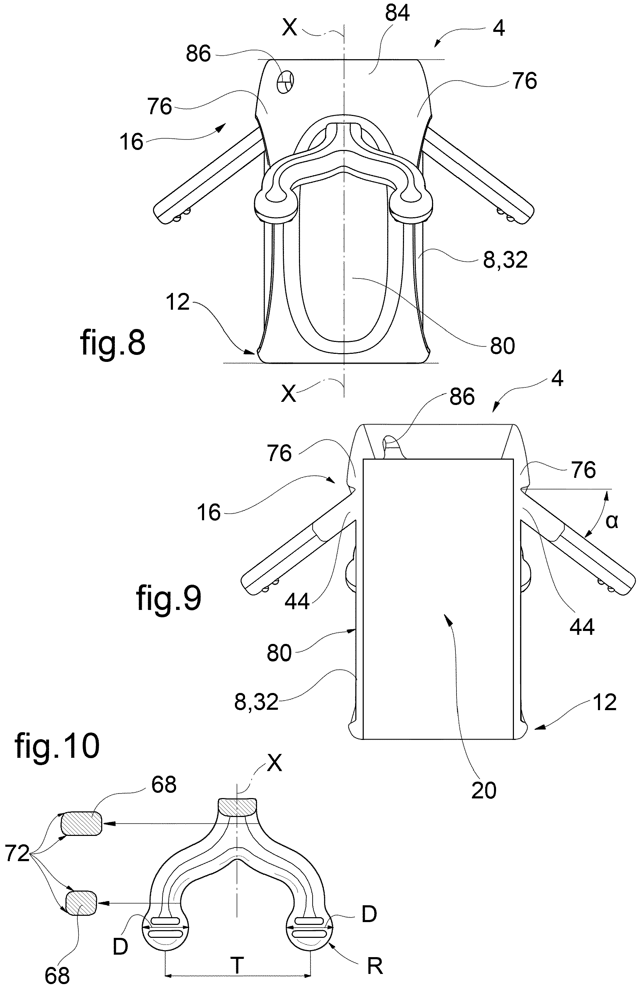

figure 8 shows a lateral view of the endoscopic medical device of figure 6;
figure 9 shows a cross-sectional view of the endoscopic medical device of figure 6;
figure 10 shows some enlarged details, even cross-sectional, of the endoscopic medical device of figure 6;

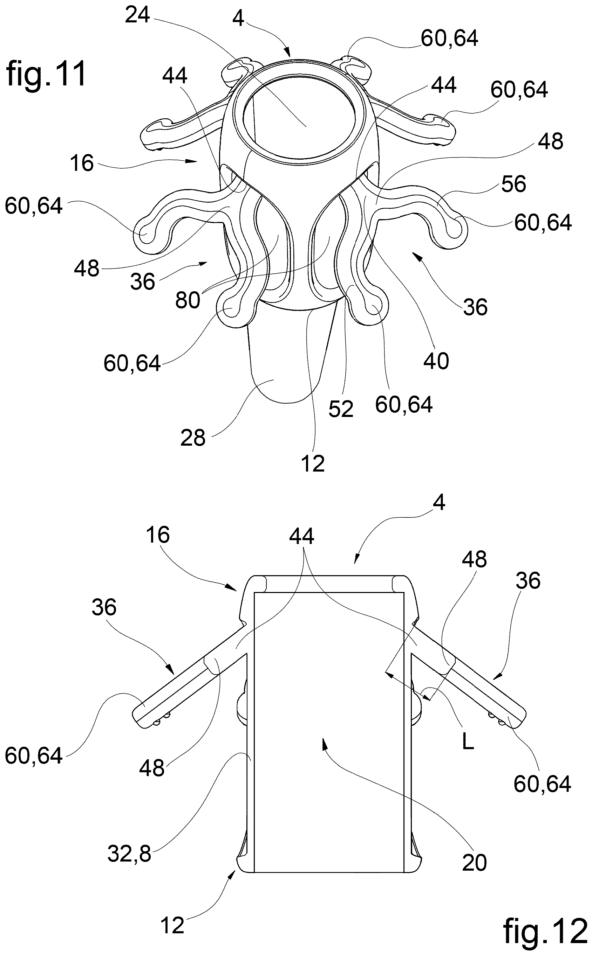

figure 11 shows a top perspective view of a colonoscopy on whose distal end there is fitted an endoscopic medical device according to a first embodiment of the present invention;
figure 12 shows a cross-sectional view of the endoscopic medical device shown in figure 11;

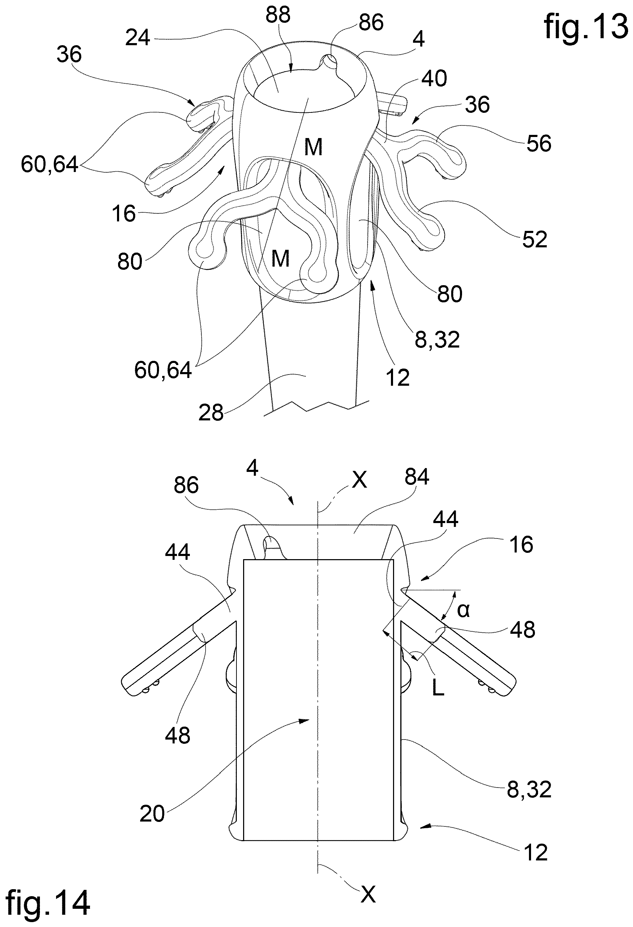

figure 13 shows a top perspective view of a colonoscopy on whose distal end there has been fitted an endoscopic medical device according to a second embodiment of the present invention;
figure 14 shows a cross-sectional view of the endoscopic medical device shown in figure 12;

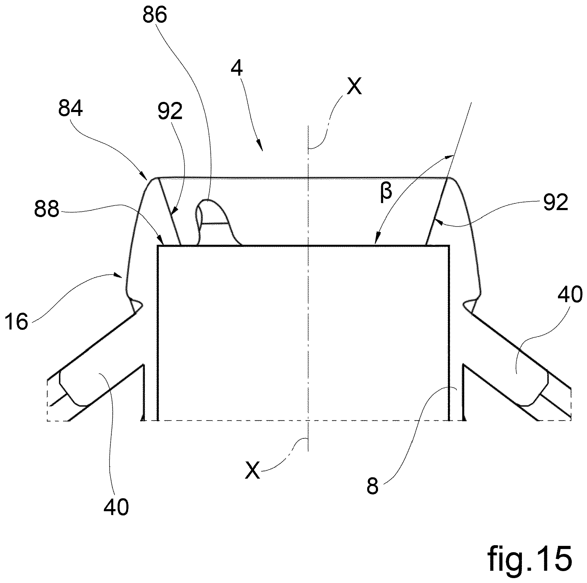

figure 15 shows a cross-sectional enlarged view of a distal portion of the endoscopic medical device according to a possible embodiment of the present invention.

The elements or components shared by the embodiments described below will be indicated using the same reference numerals.

### Detailed description

With reference to the above-mentioned figures, an overall view of an endoscopic medical device, in particular for colonoscopy, is generally indicated with 4.

In particular, the endoscopic medical device 4 comprises a main body 8, cylindrical with respect to a main extension axis X-X, which extends from a proximal end 12 to a distal end 16.

Said main body 8 is hollow so as to delimit a cavity 20 adapted to at least partially house a distal portion 24 of an endoscope 28 which can be associated therewith, on the side of said proximal end 12 of the main body 8.

The main body 8, at an outer side wall 32, opposite to said cavity 20, has a plurality of flexible fins 36 provided with arms 40 which, in a resting condition, extend along main directions Y-Y incident with said main extension axis X-X according to an opening angle α comprised between 10 and 55 degrees, measured from the side of the distal end 16.

According to a possible embodiment, said opening angle α is comprised between 20 and 45 degrees, preferably it is comprised between 35 and 39 degrees, even preferably it is equal to 37 degrees.

Said arms 40 are engaged with the outer side wall 32 at fixing ends 44 for fixing to the main body 8 and, at cantilevered ends 48, opposite to the fixing ends 44, branch into pairs of arms 52,56 arranged symmetrically with respect to a centerline plane M-M passing through said arms 40 and through said main extension axis X-X.

According to a possible embodiment, said branches 52,56, at free ends 60, opposite to opposite to the cantilevered end 48 of the arm 40, have rounded tips 64 having a determined radius of curvature R.

According to an embodiment, said radius of curvature R is comprised between 1.4 and 2.4 mm, preferably it is equal to 1.9 m.

According to an embodiment, said rounded tips 64 may be cylindrical-shaped with circular cross-section or they may be spherical.

According to a possible embodiment, 3 to 5 flexible fins 36 are provided, preferably angularly equally spaced from each other, with respect to the main extension axis X-X, along the outer side wall 32.

According to a possible embodiment, the arm 40 has a greater thickness than the thickness of said branches 52,56.

According to a possible embodiment, the arm 40 has a greater width than the width of said branches 52,56.

According to a possible embodiment, the arm 40 and the branches 52,56 have a quadrangular cross-section 68 with rounded edges 72, wherein the section is oriented so as to have a lesser moment of inertia with respect to a bending along the main extension axis X-X.

According to a possible embodiment, the arm 40 and the branches 52,56 have a constant thickness with respect to a radial cross-sectional plane parallel with and passing through the main extension axis X-X.

According to an embodiment, said thickness of the arm 40 and of the branches 52,56 is preferably smaller than a diameter D of said rounded tips 64 of the branches 52,56.

According to an embodiment, the width of the branches 52,56, and preferably also of the arm 40, is smaller than a diameter D of said rounded tips 64 of the branches 52,56.

Advantageously, the main body 8 and the flexible fins 36 are made of medical grade silicone material or a thermoplastic elastomer. Advantageously, furthermore, said material has a hardness between 40 and 60 SHORE A, preferably equal to about 50 SHORE A.

Preferably, said flexible fins 36 have an increasing flexibility moving from the fixing ends 44 to the free ends 60.

According to a possible embodiment, said arms 40 have a cantilevered length L, with respect to the fixing end 44, which are smaller than the lengths of said branches 52,56, equal to the distance between the cantilevered end 48 of the arm 40 and the free ends 60.

According to a possible embodiment, the free ends 60 of the branches 52,56, opposite to the cantilevered end 48 of the arm 40, diverge with respect to each other for a transversal distance T which is greater than the length of the arms 40.

Said transversal distance T is for example equal to a diameter of the main body 8, measured perpendicularly to the main extension axis X-X.

According to a possible embodiment, the main body 8, at the fixing ends 44 of the arms 40, on the side of the distal end 16, has bulges 76 which serve as upper stops in the rotation of the arms 40 towards the distal end 16 of the main body 8.

According to a possible embodiment, the main body 8, at the fixing ends 44 of the arms 40, on the side of the proximal end 12, has recesses 80 which serve as partial seats of the arms 40 when rotated bent towards the proximal end 12 of the main body 8.

According to a possible embodiment, the main body 8, at the distal end 16, comprises a cap 84. Said cap 84 is advantageously transparent, preferably having a height comprised between 1 and 4 mm, even more preferably equal to 2 mm, and it is particularly made of the same material as the endoscopic medical device 4. According to a possible embodiment, the cap 84 has a hole 86 preferably arranged at the lens of the endoscope 28. Said hole 86 advantageously allows the draining of the liquids cleaning the lens of the endoscope 28 from any dirt that could stop in the cap 84 during the endoscopic examination.

According to a possible embodiment, for a blocking ring 88 is provided, which is positioned on the tip or distal end 16 of the main body 8 and shaped so as to allow to correctly position the endoscopic medical device 4 on the tip of the endoscope 28. Preferably, said blocking ring 88 is positioned below said hole 86; preferably said blocking ring 88 forms an abutment or stop for the distal portion 24 of the endoscope 28 and it guarantees the correct and full insertion of the endoscopic medical device 4 on the endoscope 28.

The blocking ring 88 further advantageously allows to prevent the endoscope 28 from being positioned erroneously at the area designed for the cap 84, and therefore prevent the lens of the endoscope 28 from coming into contact with the mucous membrane.

According to a possible embodiment, the cap 84 has a flared top portion 92 having a flaring angle β greater than 95°, measured with respect to a horizontal plane perpendicular to the main extension axis X-X. Said flared top portion 92 has the advantage of not interfering with the view angle of the video camera arranged on the distal portion 24 of the endoscope 28. Furthermore, such flared top portion 92 advantageously allows to prevent the excessive bending of the cap 84 (for example if made of soft silicone material) both inwards and outwards with respect to the visual field of the endoscope 28.

Below is the functional description of the endoscopic medical device according to the present invention.

As mentioned above, the main body 8 is fitted onto the tip or distal portion 24 of an endoscope 28 to enable the introduction thereof.

When introducing into the body cavity and during the endoscopic examination, the flexible fins 36 elastically bend towards the proximal end 12 of the main body 8; in particular, the branches 52,56 bend more with respect to the arms 40 which are stiffer. Therefore, the branches 52,56 collapse flattening downwards on the endoscope. This characteristic is particularly advantageous should there be encountered stenosis or stricture of the colon, during an endoscopic examination.

This characteristic is particularly advantageous, even generally during the advancement of the endoscopic medical device 4 given that it makes the profile of the instrument narrower and therefore the instrument may advance without difficulty. The flexibility of the branches 52 and 56 also allows not to cause traumas to the patient, when retracting the endoscopic medical device 4. The retraction of the endoscope is not always linear and continuous; at times one had to advance and then return backwards again. The distal flexibility of the fins, and in particular of the branches 52,56, allows the tips 64 of the arms 40 not to pivot on the walls of the colon and therefore avoid micro traumas to the mucous membrane. Furthermore, when retracting the endoscopic medical device 4, having flexible tips means being sure not to ever apply excessive forces to the colon, given that, encountering resistance, the endoscopic medical device 4 will yield elastically instead of the anatomy of the patient yielding.

The geometric conformation of the doubled branches 52,56 allows greater stability of the medical device. The double branches 52,56 support each other, mutually supporting the overall radial opening of the medical device, preventing it from losing grip on the tissue, like it happens in the case of systems with single fins. Should a branch 52,56 slip, the remaining part binds the branch 56,52 thereto without gripping, holding it in position for a resumption of the function thereof.

Therefore, the arm 40 on the one hand allows a certain bending independence of the branches 52,56 which may therefore better adapt to the morphological conformation of the cavity section to be explored and - on the other hand - it creates a constraint between the branches 52,56 in question.

Furthermore, the apex of the oval, that is the bulging 76 of the material above the fixing end 44 of each arm 52,56 serves as a block for the arms 40 when retracting the medical device 4. In this manner, the arms 40 always remain in open position adapted to facilitate the gripping and the stretching of the colon folds, always in a non-traumatic manner.

Furthermore, the bulging 76 prevents the arms 40 of the medical device 4, when retracting the instrument, from projecting excessively forward, therefore entering into the visual field of the video camera and interfering with the endoscopic examination.

As observable in the light of the above, the present invention allows to overcome the drawbacks observed in the prior art.

In particular, the present invention allows to stretch - effectively and in a non-traumatic fashion - the folds of the colon, so as to increase the adenoma detection rate (adr) during endoscopic procedures, especially endoscopic screening.

Advantageously, the enlarged and rounded tips are non-traumatic and, at the same time, they have an improved grip so as to grip the tissues better. Furthermore, the rounded shape allows to prevent lesions to the mucous membrane.

The central body with a thicker arched structure allows to effectively support the stem, and it also facilitates the insertion of the medical device into the endoscope.

Furthermore, the device of the present invention better adapts to the morphology of the colonoscope. A more adherent fitting of the medical device ensures a better fixing thereof and ensures that the latter never inadvertently detaches from the tip of the colonoscope like it happens in some prior art solutions. The embodiment of the endoscopic medical device made of silicone material, makes the device soft and adaptable to the colonoscope. The diameters of the colonoscope most commonly used for endoscopic screening procedures are 12.8 mm and 13.2 mm. The device is made smaller in diameter with respect to these dimensions, so that it can (for example thanks to the properties of silicone) be elastic and widen fitting into the colonoscope. Once inserted, between the smaller diameter, the inherent ultra-high anti-slip property of silicone, and the absence of a rigid structure on the body of the endoscopic medical device, ensure a perfect fitting and that the endoscopic medical device can never be inadvertently detached from the tip or distal portion of the endoscope.

There can be used any material other than silicone provided it has the same properties and/or the same advantages.

Furthermore, the device of the present invention advantageously has a smaller thickness with respect to the prior art solutions: therefore, such small thickness facilitates the step for inserting and advancing the instrument into the colon section, when the arms, and in particular the branches, collapse flattening downwards on the endoscope. This characteristic is particularly advantageous should there be encountered stenosis and stricture of the colon.

Furthermore, the number of fulcrums of the arms which branch from the central body are advantageously small with respect to the prior art solutions: this allows to widen the empty spaces between the bases of the arms, allowing a very easy and effortless through-passing of fluids and stool.

Furthermore, the blocking ring positioned on the tip of the medical device allows to correctly position the medical device on the tip of the endoscope.

Furthermore, the apex and the oval, that is the bulging of the material above the fulcrum of each arm, serves as a rotary block for the arms when retracting the medical device. In this manner, the arms remain in open position adapted to facilitate the gripping and the stretching of the colon folds, always in a non-traumatic manner.

The bulging of the material above the fulcrum of the arms further prevents the arms of the device, when retracting the instrument, from projecting excessively forward, therefore entering into the visual field of the video camera, interfering with the endoscopic examination.

The geometric conformation of the double branches allows greater stability of the medical device. As a matter of fact, the double branches support each other, mutually supporting the overall radial opening of the medical device, preventing it from losing grip on the tissue, like it happens in the case of systems with single fins. The Y-shaped system therefore ensures greater stability, a greater opening and a greater effectiveness of the device especially in terms of non-traumatic stretching of the colon folds. As a matter of fact, should a part or branch of the arm slip, the remaining part binds the branch thereto without gripping, holding it in position for a resumption of the function thereof.

According to the current endoscopic techniques, in order to obtain a progressive advancement of the colonoscope one has to carry out maneuvers which provide for - continuously and alternatively - pushing the instrument, anchoring to the colon, and retracting the instrument. Advantageously, the conformation of the arms and of the branches allows greater grip and anchoring on the intestinal folds and curves during the endoscopic examination, while ensuring non-traumatic maneuver at the same time. Therefore, this also allows a faster advancement of the instrument and makes the exam shorter and less painful for the patient.

The embodiment of the device provided with a cap prevents the lens of the instrument from directly resting on the mucous membrane therefore obstructing the endoscopic read-out when moving the endoscope. Experiments showed that - in the version of the endoscopic medical device provided with cap, the latter is not framed by the video camera, in particular due to its flared shape. Therefore, this offers the benefits of a cap but without the classic visibility limitation of the cap, visibility being crucial during screening examinations.

Furthermore, the device becomes a further arm of the operator and helps the latter both to maneuver the grip on the colon and move the tissues or folds of the colon out of the way.

With the aim of meeting contingent and specific needs, a person skilled in the art may subject the solutions described above to numerous modifications and variants.

The scope of protection of the invention is defined by the claims below.

## Claims

1. Endoscopic medical device (4) comprising a main body (8), cylindrical with respect to a main extension axis (X-X), which extends from a proximal end (12) to a distal end (16),
said main body (8) being hollow so as to delimit a cavity (20) adapted to at least partially house a distal portion (24) of an endoscope (28) which can be associated therewith, on the side of said proximal end (12) of the main body (8),
said main body (8), at an outer side wall (32), opposite to said cavity (20), having a plurality of flexible fins (36) provided with arms (40) which, in a resting condition, extend along main directions (Y-Y) incident with said main extension axis (X-X) according to an opening angle (α) comprised between 10 and 55 degrees, measured from the side of the distal end (16),
wherein said arms (40) are engaged with the outer side wall (32) at fixing ends (44) for fixing to the main body (8),
said endoscopic medical device (4) being **characterized in that** said arms (40), at cantilevered ends (48) opposite to the fixing ends (44), branch into pairs of branches (52,56), arranged symmetrically with respect to a centerline plane (M-M) passing through said arms (40) and through said main extension axis (X-X).

2. Endoscopic medical device (4) according to claim 1, **characterized in that** 3 to 5 flexible fins (36) are provided along the outer side wall (32) of the main body (8), and wherein said opening angle (α) is comprised between 20 and 45 degrees, preferably it is comprised between 35 and 39 degrees, preferably it is equal to 37 degrees.

3. Endoscopic medical device (4) according to claim 1 or 2, **characterized in that** said branches (52,56), at free ends (60), opposite to the cantilevered end (48) of the arm (40), have rounded tips (64) having a determined radius of curvature (R), preferably comprised between 1.4 and 2.4 mm, more preferably equal to 1.9 mm,
wherein the width of the branches (52,56), and preferably also of the arm (40), is smaller than a diameter (D) of said rounded tips (64) of the branches (52,56).

4. Endoscopic medical device (4) according to claim 3, **characterized in that** said rounded tips (64) have a cylindrical shape with circular cross-section or they are spherical.

5. Endoscopic medical device (4) according to any one of claims 1 to 4, **characterized in that** the arms (40) have a greater width than the width of said branches (52,56).

6. Endoscopic medical device (4) according to any one of claims 1 to 5, **characterized in that** the arms (40) and the branches (52,56) have a constant thickness with respect to a radial cross-sectional plane parallel with and passing through the main extension axis (X-X).

7. Endoscopic medical device (4) according to any one of claims 1 to 6, **characterized in that** the arms (40) and the branches (52,56) have a quadrangular cross-section (68) with rounded edges (72), wherein the quadrangular cross-section (68) is oriented so as to have lesser moment of inertia with respect to a bending along the main extension axis (X-X).

8. Endoscopic medical device (4) according to any one of claims 1 to 7, **characterized in that** said flexible fins (36) have an increasing flexibility moving from the fixing ends (44) to the free ends (60).

9. Endoscopic medical device (4) according to any one of claims 1 to 8, **characterized in that** said arms (40) have a cantilevered length, with respect to the fixing end (44), which is smaller than the lengths of said branches (52,56), equal to the distance between the cantilevered end (48) of the arm (40) and the free ends (60).

10. Endoscopic medical device (4) according to any one of claims 1 to 9, **characterized in that** the free ends (60) of the branches (52,56), opposite to the cantilevered end (48) of the arm (40), diverge with respect to each other for a transversal distance (T) which is greater than the length of the arms (40) and preferably equal to a diameter of the main body (8).

11. Endoscopic medical device (4) according to any one of claims 1 to 10, **characterized in that** the main body (8), at the fixing ends of the arms, on the side of the distal end, has bulges which serve as upper stops in the rotation of the arms towards the distal end.

12. Endoscopic medical device (4) according to any one of claims 1 to 11, **characterized in that** the main body, at the ends for fixing the arms, on the side of the proximal end, has recesses which serve as partial seats of the arms when rotated or bent towards the proximal end.

13. Endoscopic medical device (4) according to any one of claims 1 to 12, **characterized in that** the main body, at the distal end, comprises a cap (84).

14. Endoscopic medical device (4) according to claim 13, **characterized in that** the cap (84) has a flared top portion (92) having a flaring angle (β) greater than 95°, said flaring angle being measured with respect to a horizontal plane perpendicular to the main extension axis (X-X).

15. Endoscopic medical device (4) according to any one of claims 1 to 14, **characterized in that** a blocking ring (88) is provided, which is positioned on the tip of the main body and shaped so as to allow to correctly position the endoscopic medical device (4) on the tip of the endoscope (28).

## Patentansprüche

1. Endoskopisches medizinisches Gerät (4), umfassend einen im Verhältnis zu einer Hauptverlaufsachse (X-X) zylindrischen Hauptkörper (8), der von einem proximalen Ende (12) zu einem distalen Ende (16) verläuft,
wobei der genannte Hauptkörper (8) hohl ist, um einen Hohlraum (20) zu begrenzen, der geeignet ist, mindestens teilweise einen distalen Abschnitt (24) eines kompatiblen Endoskops (28) von der Seite des genannten proximalen Endes (12) des Hauptkörpers (8) aufzunehmen,
wobei der genannte Hauptkörper (8) an einer dem genannten Hohlraum (20) gegenüberliegenden äußeren Seitenwand (32) eine Vielzahl von biegsamen Klappen (36) aufweist, die mit Armen (40) versehen sind, die im Ruhezustand entlang vorherrschender Richtungen (Y-Y) verlaufen, die mit der genannten Hauptverlaufsachse (X-X) gemäß einem Öffnungswinkel (α) zwischen 10 und 55 Grad, gemessen von der Seite des distalen Endes (16), inzidieren,
wobei die genannten Arme (40) sich mit der äußeren Seitenwand (32) an Befestigungsenden (44) mit dem Hauptkörper (8) im Eingriff befinden,
wobei das genannte endoskopische medizinische Gerät (4) **dadurch gekennzeichnet ist, dass** sich die genannten Arme (40), an auskragenden Enden (48) gegenüber den Befestigungsenden (44), in Paare von Zweigen (52,56) zweiteilen, die im Verhältnis zu einer durch die genannten Arme (40) und die genannte Hauptverlaufsachse (X-X) verlaufenden Mittelebene (M-M) symmetrisch angeordnet sind.

2. Endoskopisches medizinisches Gerät (4) nach Anspruch 1, **dadurch gekennzeichnet, dass** 3 bis 5 biegsame Klappen (36) entlang der äußeren Seitenwand (32) des Hauptkörpers (8) vorgesehen sind, und
bei dem der genannte Öffnungswinkel (α) zwischen 20 und 45 Grad, vorzugsweise zwischen 35 und 39 Grad, vorzugsweise 37 Grad beträgt.

3. Endoskopisches medizinisches Gerät (4) nach Anspruch 1 oder 2, **dadurch gekennzeichnet, dass** die genannten Zweige (52,56), an den freien Enden (60) gegenüber dem auskragenden Ende (48) des Arms (40), abgerundete Spitzen (64) mit einem bestimmten Krümmungsradius (R), vorzugsweise zwischen 1,4 und 2,4 mm, noch bevorzugter von 1,9 mm, aufweisen,
wobei die Breite der Zweige (52, 56), und vorzugsweise auch des Arms (40), im Verhältnis zu einem Durchmesser (D) der genannten abgerundeten Spitzen (64) der Zweige (52,56) geringer ist.

4. Endoskopisches medizinisches Gerät (4) nach Anspruch 3, wobei die genannten abgerundeten Spitzen (64) eine zylindrische Form mit kreisförmigem Querschnitt aufweisen oder kugelförmig sind.

5. Endoskopisches medizinisches Gerät (4) nach einem beliebigen der Ansprüche 1 bis 4, **dadurch gekennzeichnet, dass** die Arme (40) eine im Verhältnis zu der Breite der genannten Zweige (52,56) größere Breite aufweisen.

6. Endoskopisches medizinisches Gerät (4) nach einem beliebigen der Ansprüche 1 bis 5, **dadurch gekennzeichnet, dass** die Arme (40) und die Zweige (52,56) im Verhältnis zu einer radialen Schnittebene, die parallel zur Hauptverlaufsachse (X-X) und durch diese verläuft, eine konstante Stärke aufweisen.

7. Endoskopisches medizinisches Gerät (4) nach einem beliebigen der Ansprüche 1 bis 6, **dadurch gekennzeichnet, dass** die Arme (40) und die Zweige (52,56) einen viereckigen Abschnitt (68) mit abgerundeten Kanten (72) aufweisen, wobei der viereckige Abschnitt (68) so ausgerichtet ist, dass er im Verhältnis zu einer Biegung entlang der Hauptverlaufsachse (X-X) ein geringeres Trägheitsmoment aufweist.

8. Endoskopisches medizinisches Gerät (4) nach einem beliebigen der Ansprüche 1 bis 7, **dadurch gekennzeichnet, dass** die genannten biegsamen Klappen (36) bei ihrer Bewegung von den Befestigungsenden (44) zu den freien Enden (60) eine zunehmende Biegsamkeit aufweisen.

9. Endoskopisches medizinisches Gerät (4) nach einem beliebigen der Ansprüche 1 bis 8, **dadurch gekennzeichnet, dass** die genannten Arme (40) eine, im Verhältnis zu dem Befestigungsende (44), auskragende Länge aufweisen, die im Verhältnis zu den Längen der genannten Zweige (52,56) geringer ist und dem Abstand zwischen dem auskragenden Ende (48) des Arms (40) und den freien Enden (60) entspricht.

10. Endoskopisches medizinisches Gerät (4) nach einem beliebigen der Ansprüche 1 bis 9, **dadurch gekennzeichnet, dass** die freien Enden (60) der Zweige (52,56), die dem auskragenden Ende (48) des Arms (40) gegenüberliegen, auf einem querliegenden Abstand (T), der größer als die Länge der Arme (40) ist und vorzugsweise einem Durchmesser des Hauptkörpers (8) entspricht, auseinanderlaufen.

11. Endoskopisches medizinisches Gerät (4) nach einem beliebigen der Ansprüche 1 bis 10, **dadurch gekennzeichnet, dass** der Hauptkörper (8) an den Befestigungsenden der Arme, von der Seite des distalen Endes her, Ausbuchtungen aufweist, die bei der Drehung der Arme zu dem distalen Ende als obere Anschläge fungieren.

12. Endoskopisches medizinisches Gerät (4) nach einem beliebigen der Ansprüche 1 bis 11, **dadurch gekennzeichnet, dass** der Hauptkörper, an den Befestigungsenden der Arme, von der Seite des proximalen Endes her, Vertiefungen aufweist, die, wenn die Arme zu dem genannten proximalen Ende gedreht oder gebogen sind, als teilweise Aufnahmen der Arme fungieren.

13. Endoskopisches medizinisches Gerät (4) nach einem beliebigen der Ansprüche 1 bis 12, **dadurch gekennzeichnet, dass** der Hauptkörper an dem distalen Ende eine Kappe (84) umfasst.

14. Endoskopisches medizinisches Gerät (4) nach Anspruch 13, wobei die Kappe (84) einen aufgeweiteten oberen Abschnitt (92) mit einem Aufweitungswinkel (β) von mehr als 95° aufweist, wobei der genannte Aufweitungswinkel im Verhältnis zu einer horizontalen Ebene senkrecht zur Hauptverlaufsachse (X-X) gemessen wird.

15. Endoskopisches medizinisches Gerät (4) nach einem beliebigen der Ansprüche 1 bis 14, wobei ein Arretierring (88) vorgesehen ist, der an der Spitze des Hauptkörpers positioniert und so beschaffen ist, dass er die korrekte Positionierung des endoskopischen medizinischen Geräts (4) an der Spitze des Endoskops (28) gestattet.

## Revendications

1. Dispositif médical endoscopique (4) comprenant un corps principal (8), cylindrique par rapport à un axe d'extension principal (X-X), s'étendant d'une extrémité proximale (12) à une extrémité distale (16),
ledit corps principal (8) étant creux de manière à délimiter une cavité (20) apte à accueillir au moins partiellement une partie distale (24) d'un endoscope associable (28), du côté de ladite extrémité proximale (12) dudit corps principal (8),
ledit corps principal (8), au niveau d'une paroi latérale extérieure (32), opposée à ladite cavité (20), ayant une pluralité d'ailettes flexibles (36) pourvues de bras (40) qui, à l'état de repos, s'étendent le long de directions principales (Y-Y) incidentes audit axe d'extension principal (X-X) selon un angle d'ouverture (α) compris entre 10 et 55 degrés, mesuré du côté de l'extrémité distale (16),
dans lequel lesdits bras (40) sont imbriqués dans la paroi latérale extérieure (32) aux extrémités de fixation (44) au corps principal (8),
ledit dispositif médical endoscopique (4) étant **caractérisé en ce que** lesdits bras (40), aux extrémités en porte-à-faux (48) opposées aux extrémités de fixation (44), se ramifient en paires de ramifications (52,56), disposées symétriquement par rapport à un plan médian (M-M) passant par lesdits bras (40) et par ledit axe d'extension principal (X-X).

2. Dispositif médical endoscopique (4) selon la revendication 1, **caractérisé en ce que** 3 à 5 ailettes flexibles (36) sont prévues le long de la paroi latérale extérieure (32) du corps principal (8), et
dans lequel ledit angle d'ouverture (α) est compris entre 20 et 45 degrés, de préférence entre 35 et 39 degrés, de préférence il est égal à 37 degrés.

3. Dispositif médical endoscopique (4) selon la revendication 1 ou 2, **caractérisé en ce que** lesdites ramifications (52,56), aux extrémités libres (60) opposées à l'extrémité en porte-à-faux (48) du bras (40), présentent des pointes arrondies (64) ayant un rayon de courbure (R) déterminé, de préférence compris entre 1,4 et 2,4 mm, encore plus préférablement égal à 1,9 mm,
dans lequel la largeur des ramifications (52,56), et de préférence aussi du bras (40), est inférieure à un diamètre (D) desdites pointes arrondies (64) des ramifications (52,56).

4. Dispositif médical endoscopique (4) selon la revendication 3, dans lequel lesdites pointes arrondies (64) ont une forme cylindrique à section circulaire ou elles sont sphériques.

5. Dispositif médical endoscopique (4) selon l'une quelconque des revendications 1 à 4, **caractérisé en ce que** les bras (40) présentent une largeur supérieure à la largeur desdites ramifications (52,56).

6. Dispositif médical endoscopique (4) selon l'une quelconque des revendications 1 à 5, **caractérisé en ce que** les bras (40) et les ramifications (52,56) ont une épaisseur constante par rapport à un plan de section radiale parallèle à et passant par l'axe d'extension principal (X-X).

7. Dispositif médical endoscopique (4) selon l'une quelconque des revendications 1 à 6, **caractérisé en ce que** les bras (40) et les ramifications (52,56) présentent une section quadrangulaire (68) à bords arrondis (72), la section quadrangulaire (68) étant orientée de manière à présenter un moment d'inertie inférieur à une flexion le long de l'axe d'extension principal (X-X).

8. Dispositif médical endoscopique (4) selon l'une quelconque des revendications 1 à 7, **caractérisé en ce que** lesdites ailettes flexibles (36) présentent une flexibilité croissante en se déplaçant depuis les extrémités de fixation (44) aux extrémités libres (60).

9. Dispositif médical endoscopique (4) selon l'une quelconque des revendications 1 à 8, **caractérisé en ce que** lesdits bras (40) ont une longueur en porte-à-faux, par rapport à l'extrémité de fixation (44), inférieure aux longueurs desdites ramifications (52,56), égale à la distance entre l'extrémité en porte-à-faux (48) du bras (40) et les extrémités libres (60).

10. Dispositif médical endoscopique (4) selon l'une quelconque des revendications 1 à 9, **caractérisé en ce que** les extrémités libres (60) des ramifications (52,56), opposées à l'extrémité en porte-à-faux (48) du bras (40), s'écartent l'une de l'autre d'une distance transversale (T) supérieure à la longueur des bras (40) et de préférence égale à un diamètre du corps principal (8).

11. Dispositif médical endoscopique (4) selon l'une quelconque des revendications 1 à 10, **caractérisé en ce que** le corps principal (8), aux extrémités de fixation des bras, du côté de l'extrémité distale, présente des renflements qui servent de butées supérieures lors de la rotation des bras vers l'extrémité distale.

12. Dispositif médical endoscopique (4) selon l'une quelconque des revendications 1 à 11, **caractérisé en ce que** le corps principal, aux extrémités de fixation des bras, du côté de l'extrémité proximale, présente des creux qui servent de logements partiels des bras lors de leur rotation ou flexion vers l'extrémité proximale.

13. Dispositif médical endoscopique (4) selon l'une quelconque des revendications 1 à 12, **caractérisé en ce que** le corps principal, à l'extrémité distale, comprend un capuchon (84).

14. Dispositif médical endoscopique (4) selon la revendication 13, dans lequel le capuchon (84) présente une partie sommitale évasée (92) ayant un angle d'évasement (β) supérieur à 95°, ledit angle d'évasement étant mesuré par rapport à un plan horizontal perpendiculaire à l'axe d'extension principal (X-X).

15. Dispositif médical endoscopique (4) selon l'une quelconque des revendications 1 à 14, dans lequel un anneau de blocage (88) est prévu positionné sur la pointe du corps principal et façonné de manière à permettre au dispositif médical endoscopique (4) d'être correctement positionné sur la pointe de l'endoscope (28).
